(19) Europäisches Patentamt
European Patent Office
Office européen des brevets

(11) Veröffentlichungsnummer: **0 313 008 B1**

(12) **EUROPÄISCHE PATENTSCHRIFT**

(45) Veröffentlichungstag der Patentschrift:
08.05.91 Patentblatt 91/19

(51) Int. Cl.$^5$: **B01J 13/02**, C12N 5/00

(21) Anmeldenummer: **88117375.1**

(22) Anmeldetag: **19.10.88**

(54) **Magnetische Membrankapseln und ihre Verwendung.**

(30) Priorität: **20.10.87 DE 3735397**

(43) Veröffentlichungstag der Anmeldung:
**26.04.89 Patentblatt 89/17**

(45) Bekanntmachung des Hinweises auf die
Patenterteilung:
**08.05.91 Patentblatt 91/19**

(84) Benannte Vertragsstaaten:
**AT BE CH DE FR GB IT LI NL SE**

(56) Entgegenhaltungen:
**EP-A- 0 152 898**
**WO-A-81/00575**
**WO-A-82/00660**
**DE-A- 3 005 771**
**US-A- 4 335 094**
**US-A- 4 612 247**

(73) Patentinhaber: **HOECHST
AKTIENGESELLSCHAFT**
**Postfach 80 03 20**
**W-6230 Frankfurt am Main 80 (DE)**

(72) Erfinder: **Rüppel, Diether, Dr.**
**Karl-König-Weg 1**
**W-6230 Frankfurt am Main 80 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft magnetische Membrankapseln.

Die Benutzung von Membrankapseln hat in der Biotechnik zunehmend Bedeutung zur Aufzucht von Zellen und Mikroorganismen, insbesondere von nicht adhärenten Zellen, erlangt (vgl. US-PS 4 409 331, EP-A 0 152 898). Auch über die Verkapselung von biologischen Teilsystemen, z.B. von Enzymen wird berichtet. Bei den genannten Verfahren werden die biologischen Systeme (i.e. Zellen, Mikroorganismen, Biokatalysatoren etc.) in einer geeigneten Flüssigkeit suspendiert, die u.a. eine Komponente I enthält, die mit einer Komponente II zu einer semipermeablen biokompatiblen Membran reagieren kann. Die genannte Zellsuspension wird in Tropfenform überführt – wobei die Tropfen eine definierte Größe haben sollten – und mit einer Flüssigkeit, die die Komponente II enthält, in Kontakt gebracht. Dies führt zur Umhüllung der gebildeten Tropfen mit einer dünnen Membran.

Die erhaltenen Membrankapseln werden nun zur Anzucht bzw. Kultivierung der eingeschlossenen biologischen Systeme auf unterschiedliche Weise weiterbehandelt, u.a. werden sie mit einer Nährlösung in Kontakt gebracht, die durch die semipermeable Membran der Membrankapsel diffundiert und somit die Nährstoffversorgung der biologischen Systeme gewährleistet. Diese Art der Nährstoffversorgung birgt aber ein Problem, das mit der Größe der jeweili-gen Membrankapsel zusammenhängt ; ab einer bestimmten Größe der Kapsel diffundiert nicht mehr genügend Nährstoff in die Kapseln, alle biologischen Systeme, insbesondere die im Bereich des Mittelpunktes der Kapsel, mit Nährstoff zu versorgen, so daß ein Teil der biologischen Systeme nicht mehr wachsen kann oder sogar abstirbt (vgl. R.G. Rupp in Large-Scale Mammalian Cell Culture, Editors J. Feder und W. R. Tolbert, Academic Press, S. 19-S. 37 (1985)). Das beschriebene Problem läßt sich auch nicht lösen, indem die Kapseln nebst Nährlösung bewegt werden, z.B. durch Rühren, weil dadurch die Diffusionsverhältnisse innerhalb der Kapsel kaum verändert werden. Aus den vorbeschriebenen Gründen sollten die Durchmesser herkömmlicher Membrankapseln nicht größer als 200 bis 300 um sein (vgl. vorerwähnten Artikel von R.G. Rupp). Um die Kultivierung von biologischen Systemen wirtschaftlich zu betreiben, ist es aber von Vorteil, größere Membrankapseln zu verwenden ; die Herstellung größerer Membrankapseln, z.B. mit einen Durchmesser von mehreren Millimetern ist problemlos.

Aus den beschriebenen Gründen wäre es also wünschenswert, Mikrokapseln derart zu gestalten, daß in ihrem Innenraum ein verbesserter Nährstofftransport stattfinden kann. Diese Aufgabe wird erfindungsgemäß gelöst, indem die Membrankapseln in ihrer flüssigen Phase (Immobilisat) magnetische Partikeln enthalten, die über ein von außen angelegtes fluktuierendes Magnetfeld bewegt werden können und somit eine Möglichkeit geschaffen wird, innerhalb der Membrankapseln Flüssigkeitsbewegung zu erzeugen, die auch den Stofftransport innerhalb der Membrankapsel erhöht. Durch die Bewegung der magnetischen Partikeln werden die in der Membrankapsel miteingeschlossenen biologischen Systeme nicht geschädigt, was überraschend ist, weil man erwarten mußte, daß die magnetischen Partikeln bei ihrer Bewegung die Zellwände der biologischen Systeme zerstören.

Die vorliegende Erfindung betrifft daher Membrankapseln, bestehend aus einer kugelförmigen, semipermeablen Membran, durch die eine immobile flüssige Phase eingeschlossen ist, die dadurch gekennzeichnet sind, daß die immobile flüssige Phase magnetische Partikeln enthält. Mit dem Begriff "magnetische Partikeln" sind in diesem Zusammenhang insbesondere ferromagnetische Partikeln gemeint. Neben dem gegenüber anderen Membrankapseln verbesserten Stofftransport innerhalb der Kapseln haben die erfindungsgemäßen Membrankapseln noch weitere Vorteile, die hauptsächlich durch die Möglichkeit begründet sind, die gesamte Kapsel durch Anlegen eines äußeren Magnetfeldes zu bewegen bzw. zu fixieren. Diese Vorteile sind bereits von anderen Partikeln bekannt, die ferromagnetische Kristallite enthalten, sich aber von den erfindungsgemäßen Membrankapseln durch ihre quasi Festkörperstruktur ohne flüssige Phase unterscheiden (vgl. DE-OS 34 44 939, EP-B 0 016 552 und EP-A 0 206 077).

Mit den erfindungsgemäßen Membrankapseln können fast beliebige Stoffe zusammen mit den magnetischen Partikeln enkapsuliert werden. Bevorzugt ist die Enkapsulierung von biologisch aktivem Material wie z.B. lebenden Zellen, Mikroorganismen, Enzymen, Hormonen, Antikörpern und/oder Biokatalysatoren, jeweils in einem geeigneten Medium. Besonders bevorzugt sind antikörperproduzierende Zellen wie z.B. Hybridomazellen, Pflanzenzellen wie z.B. Nutzpflanzenzellen, Bakterienzellen wie z.B. E. coli und Milchsäurebakterien, Streptomyceten, Pilze wie z.B. Penicillinium und Aspergillus und Hefen wie z.B. Weinhefe.

Weiterhin werden Flüssigkeiten mitenkapsuliert, in denen die biologisch aktiven Materialien suspendiert bzw. gelöst sind. Diese Stoffe stammen z.B. aus der Gruppe der Lösungsmittel, wie z.B. Wasser oder Glycerin, denen ggf. noch Zusatzstoffe wie z.B. Nährmittel und Spurenelemente etc. zugegeben worden sind.

Die magnetischen Partikeln können aus unterschiedlichen Materialien bestehen. Sie sollten in der Flüssigkeit, in der die zu enkapsulierenden Substanzen suspendiert bzw. gelöst werden, weitgehend unlöslich sein und sie sollten mit den Substanzen verträglich sein ; d.h. z.B. für lebende Zellen und Mikroorganismen sollten sie nicht toxisch sein. Bevorzugt sind Verbindungen, die Eisen, Kobalt oder Nickel enthalten. Besonders bevorzugt sind die Eisenoxide Hämatit und Magnetit, die auf literaturbekannte Weise hergestellt werden können (vgl. z.B. Ullmanns Encyklopädie der technischen Chemie, 3. Aufl.6. Band, S. 420 ff., Urban & Schwarzenberg, München, Berlin (1955)) und auch Handelspräparate sind, wie z.B. das Produkt ®Bayferrox 8600 der Firma Bayer AG, Leverkusen.

Die Größe der zu enkapsulierenden magnetischen Partikeln kann einen weiten Bereich überstreichen. Bevorzugt sind Partikeln mit einem maximalen Durchmesser zwischen ca. 0,01 und 15 µm, besonders bevorzugt zwischen ca. 0,05 und 10 µm, insbesondere zwischen ca. 0,1 und 2 µm. Die Partikeln mit den jeweiligen Größenverteilungen kann man z.B. durch "fraktioniertes Sieben" mit entsprechenden Sieben, erhalten.

Es ist sinnvoll, die Membrankapseln mit einem definierten Gewichtsanteil an magnetischen Partikeln zu versehen. Vorteilhaft ist ein gewichtsprozentualer Anteil an der Kapsel von ca. 0,01 bis 5%, besonders vorteilhaft von ca. 0,02 bis 3%, insbesondere von ca. 0,03 bis 1%. Ein hoher prozentualer Anteil an magnetischen Partikeln hat den Vorteil, daß die Membrankapseln besonders leicht mit Magnetfeldern bewegt bzw. fixiert werden können und daß die Sedimentation der Membrankapseln aufgrund ihres hohen spezifischen Gewichts besonders leicht erfolgt. Der Nachteil eines hohen Gehalts an magnetischem Material liegt in der starken mechanischen Belastung der Kapselmembran durch die auftretenden Gravitations- und Magnetkräfte.

Die Membrankapseln, die erfindungsgemäß mit den magnetischen Partikeln ausgestattet werden, können eine Größe haben, die über einen weiten Bereich variiert. Besonders geeignet sind Kapseln mit einem Durchmesser zwischen ca. 0,05 und 7 mm, insbesondere zwischen 0,1 und 5 mm. Besonders bevorzugt ist ein Kapseldurchmesser zwischen ca. 0,5 und 3 mm.

Die Membran der Kapseln, die erfindungsgemäß mit magnetischen Partikeln ausgestattet werden, kann auf unterschiedliche Weise hergestellt werden. Bevorzugt ist eine Verkapselung, wie in der EP-A 0 173 915 beschrieben, auf der Basis von Polysacchariden, die durch Inkontaktbringen mit mehrwertigen Ionen vernetzt werden ; besonders bevorzugt ist eine Verkapselung aus Alginat, das durch Calcium vernetzt ist. Ein anderes bevorzugtes Verkapselungsmaterial besteht aus Polyelektrolytkomplexen, wie z.B. in der deutschen Anmeldung mit dem Aktenzeichen P 37 29 434 beschrieben. Bei diesem Membranmaterial wird eine Polysäure zusammen mit dem zu verkapselnden Material vertropft und der entstehende Tropfen wird mit einer Polybase in Kontakt gebracht, wobei die Membran sich ausbildet. Besonders bevorzugte Polysäuren sind z.B. Alginat, Carrageen, Carboxymethylcellulose oder Xanthan. Besonders geeignete Polybasen sind z.B. Allyl- und Methallylamine, N-Vinylimidazol und N-Vinylmethylimidazol, die gegebenenfalls auch noch durch Kopolymere, wie z.B. N-Vinylpyrrolidon, verknüpft sein können. Weitere bevorzugte Polysäuren und -basen sind diejenigen, die auf Acrylbasis aufgebaut sind, wie z.B. in der EP-A 0 188 309 beschrieben.

Die eigentliche Herstellung der Membrankapseln erfolgt, wie bereits erläutert, indem die zu verkapselnde Flüssigkeit, in der der aktive Stoff und die magnetischen Partikeln suspendiert bzw. gelöst sind, vertropft wird und anschließend durch die Einwirkung einer zweiten Komponente die Kapselmembran gebildet wird. Zur Vertropfung der jeweiligen Suspension können beliebige bekannte Vertropfungseinrichtungen benutzt werden. Zu bevorzugen ist eine Vertropfungseinrichtung, bei der die zu vertropfende Suspension aus einer Kapillare austritt, während an der Kapillaröffnung ein umhüllender Gasstrom in Richtung der Kapillaröffnung strömt, der die Tropfen abdrückt. Eine derartige Vertropfungseinrichtung ist in F. Lim ed., "Biomedical Applications of Microencapsulation", CRC Press, 1984, beschrieben.

Die Manipulation der erfindungsgemäß magnetische Partikeln enthaltenden Membrankapseln mittels Magnetfelder kann auf unterschiedliche Weise erfolgen. Die weitaus wichtigste Manipulation, das Rühren innerhalb der Kapsel, kann durchgeführt werden, indem ein fluktuierendes Magnetfeld angelegt wird, z.B. durch Einsatz eines Magnetrührers. Die optimale Stärke und Frequenz des jeweiligen Magnetfeldes ist von der Größe der Membrankapseln, von der Größe der magnetischen Partikeln und von der Konzentration der magnetischen Partikeln abhängig. Die optimale Fluktuationsfrequenz ist für die jeweiligen Arbeitsbedingungen empirisch zu ermitteln, was problemlos mittels mikroskopischer Beobachtung möglich ist. Unterhalb einer kritischen Grenzfrequenz drehen sich die Partikeln im Inneren der Kapsel und sorgen für eine gute Durchmischung. Bei genügend großer Viskosität im Inneren der Kapsel führen diese dann noch eine Eigendrehung aus, was auf schonende Weise die Entstehung einer Diffusionsverarmungsschicht außerhalb der Membrankapseln verhindert.

Weiterhin bietet der Magnetismus der Kapseln als Gesamtheit die Möglichkeit, die Kapseln durch Anlegen eines Magnetfeldes zu bewegen oder zu fixieren. Besonders bewährt hat sich die Möglichkeit, die – durch den Gehalt an Teilchen hoher Dichte ohnehin schon beschleunigte – Sedimentation der Membrankapseln zu verstärken, indem am jeweiligen Gefäßboden ein Magnetfeld angelegt wird. Ein Wechsel des Nährmediums sowie Wasch- und Spülvorgänge werden durch diese Maßnahme erleichtert. Eine weitere Manipulationsmöglichkeit ist dadurch gegeben, daß die magnetischen Membrankapseln durch die Einwirkung von Magnetfeldern bewegt werden können – z.B. mit einem magnetischen Transportband – was die Durchführung automatisierter Prozesse erleichtern kann.

Die erfindungsgemäßen Membrankapseln mit einem Gehalt an magnetischen Partikeln können – wie bereits angedeutetvielfältig verwendet werden. z.B. eignen sie sich zur Aufzucht und zur Kultivierung von lebenden biologischen Systemen wie z.B. Mikroorganismen oder Zellen. Eine andere mögliche Anwendung ist die Herstellung von Produkten mittels biologisch aktiver Systeme und Verbindungen, wie z.B. mittels Mikroorganismen, Zellen, Enzymen, Biokatalysatoren usw.

Durch die nachfolgenden Beispiele soll die Erfindung näher erläutert werden.

## Beispiel 1

Hybridomazellen liegen in Dulbeccos Medium mit foetalem Kälberserum vor. Sie werden 1 : 3 mit einer 1,1%igen Methylcellulose (®Culminal 12000, Hersteller Fa. Henkel KGaA Düsseldorf) in physiologischer Kochsalzlösung gemischt. Die Methylcelluloselösung enthält 0,3 Gew.-% Hämatit (Bayferrox 8610) und 1,5 Gew.-% CaCl$_2$.

Die Vertropfung erfolgt über eine spezielle Vertropfungseinrichtung, bei der die erhaltene Suspension durch eine Kapillare mit einem Durchmesser von 0,7 mm gepreßt wird und an deren Öffnung die Tropfen von einem koaxial strömenden Luftstrom abgedrückt werden.

Die Tropfen fallen in eine physiologische Kochsalzlösung mit 0,7% Alginat. Wegen der erhöhten Dichte passieren die Tropfen ohne Probleme die Grenzfläche zur Luft und werden durch Rühren in das Reaktionsvolumen geschafft. Das ausdiffundierende Calzium komplexiert mit Alginat zu einer Membrankapsel von etwa 4 mm Durchmesser. Es schließen sich noch Waschvorgänge in physiologischer Kochsalzlösung und in einer 2%igen CaCl$_2$-Lösung an. Die Kapseln sedimentieren stets sehr schnell, insbesondere bei Einwirkung eines Magnetfeldes am Gefäßboden. Schließlich werden sie in Dulbeccos Nährmedium überführt.

Die wie zuvor beschrieben hergestellten Kapseln können durch einen Magneten leicht festgehalten werden, was den Wechsel des verbrauchten Mediums sehr erleichtert. Ein Magnetfeld richtet die magnetischen Partikeln im Inneren der Kapsel aus (s. Photo), so daß über einen Magnetrührer auch das Innere der Kapsel bewegt werden kann.

## Beispiel 2

Eine Suspension von Hybridomazellen wird mit einer 4%igen Carrageenlösung (Hersteller Fa. Sigma Chemie GmbH, München) in Dulbecco's Medium 1 : 1 (Massenverhältnis) verdünnt. Der Suspension werden 0,1% Hämatit (Bayferrox 8600) zugesetzt. Die Zellsuspension wird wie in Beispiel 1 beschrieben vertropft, wobei eine Kapillare mit 0,14 mm Durchmesser verwendet wird. Die Tropfen fallen in eine 2%ige Lösung eines Vinylmethylimidazol-Vinylpyrrolidon-Kopolymeren, das wie folgt hergestellt wird :

In einem 4 Liter-Glaskolben werden 1443 g (10 Mol) 1-Vinyl-3-methylimidazoliumchlorid und 56 g (0,5 Mol) 1-Vinyl-2-pyrrolidon in 3,8 l Wasser gelöst, das 38 g Kaliumperoxodisulfat als Initiator enthält. Der Ansatz wird 6 Stunden bei 60°C unter Stickstoff polymerisiert. Man erhält eine klare gelb-braune 40%ige Lösung mit neutralem pH-Wert. Der K-Wert beträgt 60.

Die wie zuvor beschrieben hergestellten Kapseln haben einen Durchmesser von 600 µm.

## Beispiel 3

Die nach Beispiel 2 hergestellten Kapseln werden 15 Tage in Dulbecco's Nährlösung kultiviert. Während dieser 15 Tage werden die Kapseln durch ein fluktuierendes Magnetfeld, das durch einen Magnetrührer erzeugt wird, beeinflußt. Die von den Hybridomazellen produzierten Antikörper können aus der entnommenen Kulturlösung isoliert werden. Die Syntheseleistung für monoklonale Antikörper ist nach 6 Tagen 200 ng Immunglobulin pro Kapsel und Tag.

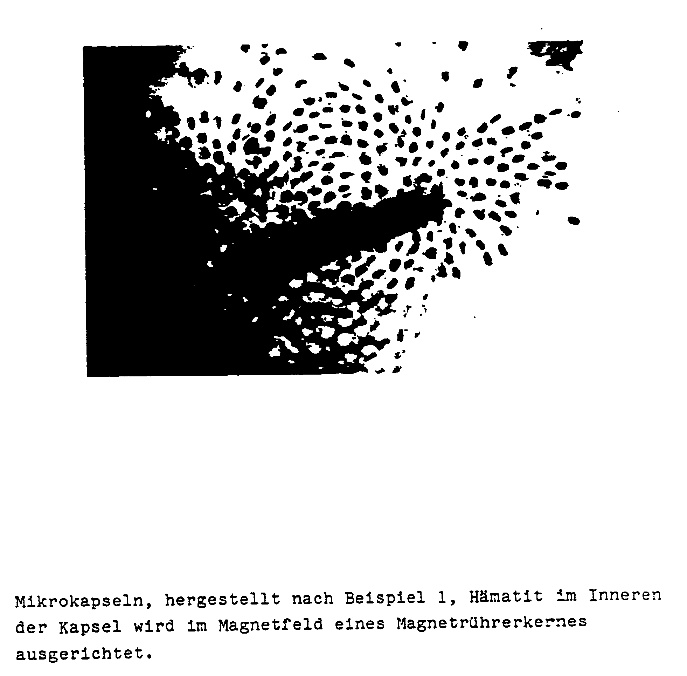

## Ansprüche

1. Membrankapseln bestehend aus einer kugelförmigen semipermeablen Membran durch die eine immobile flüssige Phase eingeschlossen ist, dadurch gekennzeichnet, daß die immobile flüssige Phase magnetische Partikel enthält.

2. Membrankapseln nach Anspruch 1, dadurch gekennzeichnet, daß die immobile Phase Enzyme, lebende Zellen oder Mikroorganismen, insbesondere Hybridomazellen enthält.

3. Membrankapseln nach Anspruch 1 oder 2, dadurch gekennzeichnet, daß die magnetischen Partikeln die chemischen Elemente Eisen und/oder Nickel und/oder Kobalt enthalten.

4. Membrankapseln nach einem oder mehreren der Ansprüche 1 bis 3, dadurch gekennzeichnet, daß die magnetischen Partikeln aus Hämatit oder Magnetit bestehen.

5. Membrankapseln nach einem oder mehreren der Ansprüche 1 bis 4, dadurch gekennzeichnet, daß die maximalen Partikeldurchmesser der magnetischen Partikeln ca. 0,01 bis 15 µm betragen.

6. Membrankapseln nach einem oder mehreren der Ansprüche 1 bis 5, dadurch gekennzeichnet, daß der gewichtsprozentuale Anteil der magnetischen Partikeln an der Kapsel mit Inhalt zwischen ca. 0,01 und 5% liegt.

7. Membrankapseln nach einem oder mehreren der Ansprüche 1 bis 6, dadurch gekennzeichnet, daß ihr Durchmesser zwischen ca. 0,05 und 7 mm liegt.

8. Membrankapseln nach einem oder mehreren der Ansprüche 1 bis 7, dadurch gekennzeichnet, daß das Membranmaterial aus Polysacchariden besteht, die durch mehrwertige Ionen vernetzt werden.

9. Membrankapseln nach einem oder mehreren der Ansprüche 1 bis 7, dadurch gekennzeichnet, daß das Membranmaterial durch die Reaktion mindestens einer Polysäure mit mindestens einer Polybase gebildet wird.

10. Membrankapseln nach Anspruch 9, dadurch gekennzeichnet, daß als Polysäuren Polysaccharide und als Polybasen Polymere auf Polyvinylbasis, insbesondere Allylamin, Methallylamin, N-Vinylimidazol und N-Vinylmethylimidazol, die mit weiteren Kopolymeren verknüpft sein können, verwendet werden.

11. Membrankapseln nach Anspruch 9, dadurch gekennzeichnet, daß als Polysäuren und Polybasen solche auf Acrylbasis verwendet werden.

12. Verwendung von Membrankapseln gemäß einem oder mehreren der Ansprüche 1-11 zur Aufzucht oder Kultivierung von Mikroorganismen oder Zellkulturen.

13. Verwendung von Membrankapseln gemäß einem oder mehreren der Ansprüche 1-11 zur Herstellung von Produkten mittels Mikroorganismen, Zellen, Biokatalysatoren oder Enzymen.

## Claims

1. A membrane capsule consisting of a globular semi-permeable membrane by means of which an immobile liquid phase is enclosed, wherein the immobile liquid phase contains magnetic particles.

2. A membrane capsule as claimed in claim 1, wherein the immobile phase contains enzymes, living cells or microorganisms, in particular hybridoma cells.

3. A membrane capsule as claimed in claim 1 or 2, wherein the magnetic particles contain the chemical elements iron and/or nickel and/or cobalt.

4. A membrane capsule as claimed in one or more of claims 1 to 3, wherein the magnetic particles comprise hematite or magnetite.

5. A membrane capsule as claimed in one or more of claims 1 to 4, wherein the maximum particle diameter of the magnetic particles is about 0.01 to 15 µm.

6. A membrane capsule as claimed in one or more of claims 1 to 5, wherein the percentage content by weight of the magnetic particles in the capsule is between about 0.01 and 5%.

7. A membrane capsule as claimed in one or more of claims 1 to 6, wherein the diameter thereof is between about 0.05 and 7 mm.

8. A membrane capsule as claimed in one or more of claims 1 to 7, wherein the membrane material comprises polysaccharides which are crosslinked by polyvalent ions.

9. A membrane capsule as claimed in one or more of claims 1 to 7, wherein the membrane material is formed by the reaction of at least one polyacid with at least one polybase.

10. A membrane capsule as claimed in claim 9, wherein polysaccharides are used as polyacids and polyvinyl based polymers, in particular allylamine, methallylamine, N-vinylimidazole and N-vinylmethylimidazole, which can be linked with additional copolymers, are used as polybases.

11. A membrane capsule as claimed in claim 9, wherein, as polyacids and polybases, those which are used are acrylic-based.

12. The use of membrane capsules as claimed in one or more of claims 1-11 for the raising or culturing of microorganisms or cell cultures.

13. The use of membrane capsules as claimed in one or more of claims 1-11 for the production of products using microorganisms, cells, biocatalysts or enzymes.

## Revendications

1. Capsules membraneuses constituées d'une membrane semi-perméable sphérique au moyen de laquelle est incluse une phase liquide immobile, caractérisées en ce que la phase liquide immobile contient des particules magnétiques.

2. Capsules membraneuses selon la revendication 1, caractérisées en ce que la phase immobile contient des enzymes, des cellules vivantes ou des micro-organismes, en particulier des cellules d'hybridomes.

3. Capsules membraneuses selon la revendication 1 ou 2, caractérisées en ce que les particules magnétiques contiennent les éléments chimiques fer et/ou nickel et/ou cobalt.

4. Capsules membraneuses selon une ou plusieurs des revendications 1 à 3, caractérisées en ce que les particules magnétiques sont constituées d'hématite ou de magnétite.

5. Capsules membraneuses selon une ou plusieurs des revendications 1 à 4, caractérisées en ce que les diamètres maximaux de particules des particules magnétiques vont d'environ 0,01 à 15 µm.

6. Capsules membraneuses selon une ou plusieurs des revendications 1 à 5, caractérisées en ce que la proportion en pourcentage en poids des particules magnétiques, par rapport à la capsule avec son contenu, est comprise entre environ 0,01 et 5%.

7. Capsules membraneuses selon une ou plusieurs des revendications 1 à 6, caractérisées en ce que leur diamètre est compris entre environ 0,05 et 7 mm.

8. Capsules membraneuses selon une ou plusieurs des revendications 1 à 7, caractérisées en ce que le matériau de membrane est constitué de polysaccharides qui sont réticulés par des ions plurivalents.

9. Capsules membraneuses selon une ou plusieurs des revendications 1 à 7, caractérisées en ce, que le matériau de membrane est formé par la réaction d'au moins un polyacide avec au moins une polybase.

10. Capsules membraneuses selon la revendication 9, caractérisées en ce que l'on utilise en tant que polyacides des polysaccharides, et, en tant que Polybases, des polymères à base polyvinylique, en particulier l'allylamine, la méthallylamine, le N-vinylimidazole et le N-vinylméthylimidazole, qui peuvent être liés à d'autres copolymères.

11. Capsules membraneuses selon la revendication 9, caractérisées en ce que l'on utilise en tant que polyacides et polybases ceux à base acrylique.

12. Utilisation de capsules membraneuses selon une ou plusieurs des revendications 1 à 11, pour la culture de micro-organismes ou de cellules.

13. Utilisation de capsules membraneuses selon une ou plusieurs des revendications 1 à 11, pour la préparation de produits au moyen de micro-organismes, de cellules, de bio-catalyseurs ou d'enzymes.

Mikrokapseln, hergestellt nach Beispiel 1, Hämatit im Inneren
der Kapsel wird im Magnetfeld eines Magnetrührerkernes
ausgerichtet.